# EUROPEAN PATENT SPECIFICATION

(11) **EP 2 994 136 B1**
(45) Date of publication and mention of the grant of the patent: **18.11.2020**
(21) Application number: 14795047.1
(22) Date of filing: 09.05.2014
(51) Int. Cl.: A61K 9/00, A61K 9/20, A61K 31/46, A61K 47/58, A61P 25/34

(54) **NICOTINE LOZENGE FORMULATION**
REZEPTUR FÜR NIKOTINPASTILLEN
FORMULATION DE PASTILLE À LA NICOTINE

(30) Priority: 10.05.2013 IN 1398DE2013
(43) Date of publication of application: 16.03.2016
(73) Proprietor: GlaxoSmithKline LLC, Wilmington, DE 19808 (US)
(72) Inventor: DIPALI, Satish, Ramchandra, Parsippany, NJ 07054 (US); NARANG, Sumeet, Bindra, Gurgaon Haryana (IN); PATHAN, Shadab, Ahmad, Gurgaon Haryana (IN)
(74) Representative: Thompson, Clive Beresford
(86) International application number: PCT/US2014/037421
(87) International publication number: WO 2014/182983

(56) References cited:
- WO-A1-2012/154563
- US-A- 5 135 753
- US-A1- 2008 260 836
- US-A1- 2011 110 880
- US-A1- 2011 110 880
- US-A1- 2013 101 652
- Anonymous: "METOLOSE (Methylcellulose USP & Hypromellose USP)", , 2005, pages 1-12, XP055303260, internet Retrieved from the Internet: URL:http://www.metolose.ru/files/metolose. pdf [retrieved on 2016-09-16]
- Anonymous: "NICORETTE-nicotine polacrilex lozenge", , June 2006 (2006-06), pages 1-25, XP055303669, internet Retrieved from the Internet: URL:https://dailymed.nlm.nih.gov/dailymed/ getFile.cfm?setid=991704ed-781a-489b-8b56- 0b558e8fc385&type=pdf&name=991704ed-781a-4 89b-8b56-0b558e8fc385 [retrieved on 2016-09-19]
- Anonymous: "Commit Lozenges Review and Results - Do Commit Lozenges Work?", , 2016, pages 1-2, XP5303525, internet Retrieved from the Internet: URL:http://www.stopsmoking.net/commit-loze nges.html [retrieved on 2016-09-19]

## Description

### TECHNOLOGY FIELD

Aspects of the present invention are directed to nicotine replacement therapy products, and, in particular, nicotine containing oral lozenges.

### BACKGROUND

It is generally known that smoking of tobacco products, such as cigarettes, cigars and pipe tobacco presents serious health risks to the user and those subjected to secondary smoke. It is also known that the use of smokeless forms of tobacco, such as chewing tobacco, spit tobacco and snuff tobacco, presents serious health risks to the user. Furthermore, the use of tobacco products in public areas is increasingly either restricted or socially unacceptable. Consequently, smokers and other tobacco users often try to quit the potentially deadly habit. Others may be forced to cut back on the amount of tobacco used as employment and social settings increasingly restrict smoking and other tobacco use.

Although the damaging effects of tobacco usage are well known, most individuals who are nicotine dependent have great difficulty overcoming their dependence on nicotine, typically in cigarette form. The difficulty arises in part due to the highly addictive nature of nicotine and the strong nicotine withdrawal symptoms that can occur when one begins to deprive the body of the nicotine to which it has grown dependent. Indeed, overcoming nicotine withdrawal symptoms is a critical challenge for those attempting to conquer nicotine dependence.

Nicotine withdrawal symptoms, particularly nicotine cravings, may arise in several ways. For instance, studies have shown that following a quit attempt, smokers report moderate levels of steady nicotine craving throughout the day. This craving can prove too much for some, leading to relapse and a return to tobacco usage for some of those individuals attempting to quit. In addition to steady cravings, smokers may also experience episodic, or acute, cravings. These acute cravings may be provoked by a number of stimuli, such as exposure to smoking related cues, seeing smoking paraphernalia, being in proximity to others engaged in smoking, or inhaling second hand smoke. Such episodic cravings may also lead to relapse if effective coping measures are not employed by the individual.

In an attempt to assist those who wish to eliminate or reduce tobacco usage, efforts have been made to provide those in need with some level of nicotine craving relief. Historically, these efforts have focused on the activity and administration of nicotine itself. This nicotine replacement therapy (NRT) helps to combat the intense nicotine withdrawal symptoms encountered by many individuals upon quitting smoking or other tobacco usage. In recent years, NRT has been successfully commercialized in both the United States and elsewhere. Such commercial NRT offerings include nicotine gums (e.g. NICORETTE® brand gums sold in the United States by GlaxoSmithKline Consumer Healthcare) and nicotine transdermal patches (e.g., NICODERM® brand patches sold by GlaxoSmithKline Consumer Healthcare).

In addition to traditional gums and patch NRT offerings, more recently, nicotine containing lozenges have been introduced commercially both within and outside the United States. For example, NICORETTE®, brand lozenges offer individuals an alternative form of NRT. US Patent 5,110,605 to Acharya et al. relates to lozenge compositions which comprise polycarbophil and alginic acid components. Other examples of nicotine containing lozenge formulations are found in a number of publications, including but not limited to, U.S. Patent 4,967,773 to Shaw; U.S. Patent 5,549,906 to Santus; U.S. Patent 6,183,775 to Ventouras; and WO 2007/104575 to Axelsson et al. Similarly, U.S. Patents 5,593,684; 5,721,257 and 5,362,496 (all to Baker et al.) disclose methods and therapeutic systems for smoking cessation, utilizing both transdermal nicotine delivery for obtaining baseline nicotine plasma levels, and transmucosal administration of nicotine to satisfy transient cravings.

Although NRT products, including nicotine containing lozenges, have gained public acceptance in many Western markets, there are still some obstacles to providing these products to many other areas of the globe. Some of these drawbacks include, for example, the high cost to manufacture NRT products and their lack of stability in more cost effective packaging options. For example, naturally occurring polymers are used in some lozenges, which adds cost to the products and makes it difficult to sell these products in less affluent markets. In addition to the costs, these polymers may also negatively impact nicotine availability by interfering with the nicotine active, especially when the nicotine active is in a resin complex, such as, for example, nicotine polacrilex. Traditional polymers may also bind with buffers in the dosage form, resulting in a slower release of nicotine. Furthermore, when using less expensive packaging, traditional lozenges lack stability for an acceptable period of time.

Accordingly, a NRT lozenge that eliminates or reduces some or all of the above-mentioned drawbacks of current NRT lozenges would be highly desirable.

### SUMMARY

The present invention is directed to a nicotine lozenge for oral administration comprising nicotine polacrilex; at least one high viscosity, water soluble, non-ionic, synthetic or semi-synthetic polymer which is an alkylcellulose polymer having a viscosity of between about 2,000 cps to about 6,000 cps as measured by Brookfield type LV Model; and at least one low viscosity, water soluble, non-ionic, synthetic or semi-synthetic alkylcellulose polymer having a viscosity of between about 50 cps and about 150 cps as measured by Capillary Viscosimeter Methods 911.

Another embodiment of the present invention are directed to a nicotine lozenge for oral administration comprising an intragranular component comprising a water soluble, non-ionic, synthetic or semi-synthetic polymer; and an extragranular component comprising nicotine polacrilex and at least one high viscosity water soluble, non-ionic, synthetic or semi-synthetic alkylcellulose polymer which is an alkylcellulose polymer having a viscosity of between about 2,000 cps to about 6,000 cps as measured by Brookfield type LV Model, and at least one low viscosity water soluble, non-ionic, synthetic or semi-synthetic alkylcellulose polymer having a viscosity of between about 50 cps and about 150 cps as measured by Capillary Viscosimeter Methods 911.

In further embodiments, lozenges of the present invention have an *in vitro* dissolution profile (as determined by USP Type I apparatus, basket, Phosphate buffer at pH 7.4, 37 °C set at rotating speed of 100rpm) of:
25 to 50% at 1 hour;
50 to 99% at 3 hours;
75 to 100% at 6 hours.

In one embodiment, lozenges of the present invention comprise a median time to maximum plasma concentration of nicotine (T*ₘₐₓ*) from about 1.2 hours to about 2 hours after administration.

In another embodiment, lozenges of the present invention comprise a mean plasma concentration (C*ₘₐₓ*) of nicotine from about 16 ng/ml to about 20 ng /ml, based on administration. In yet another embodiment, lozenges of the present invention comprise a mean plasma concentration (C*ₘₐₓ*) of nicotine between 80% and 125% of the mean plasma concentration (C*ₘₐₓ*) of 18.67 ng /ml.

In one embodiment, lozenges of the present invention provide a mean Area Under the Curve (AUC₀₋₁₂) of nicotine of between 80 and 100 ng*hr/mL. In yet another embodiment, lozenges of the present invention provide a mean Area Under the Curve (AUC₍₀₋₁₂₎) of nicotine of between 80% and 125% of the mean Area Under the Curve (AUC₍₀₋₁₂₎) of 90 ng*hr/mL.

In a further embodiment, lozenges of the present invention have a stability at a temperature of 40°C and a relative humidity of 75% in a Duplex package of at least 6 months, or at least 12 months, or at least 24 months.

### BRIEF DESCRIPTION OF DRAWINGS

Figure 1 shows compositions of embodiments of the present invention;
Figure 2 shows an exemplary process for making lozenges of the present invention;
Figure 3 shows dissolution rates of 3 embodiments of the present invention compared to a NICORETTE® Original 4mg lozenge as a reference;
Figure 4 shows the dissolution profile of three embodiments of the present invention compared to a NICORETTE® Original 4mg lozenge as a reference;
Figure 5 shows plasma concentration of 3 embodiments of the present invention compared to a NICORETTE® Original 4mg lozenge as a reference;
Figure 6 shows baseline adjusted nicotine plasma pharmacokinetic variables for 3 embodiments of the present invention compared to a NICORETTE® Original 4mg lozenge as a reference;
Figure 7 shows a statistical analysis of baseline adjusted nicotine plasma pharmacokinetic variables for 3 embodiments of the present invention compared to a NICORETTE® Original 4mg lozenge as a reference;
Figure 8 shows a table of various packaging options utilized for stability testing of embodiments of the present invention;
Figure 9 shows an analysis of impurity levels of 3 embodiments of the present invention over various time periods in an ALU/ALU package type;
Figure 10 shows an analysis of impurity levels of 3 embodiments of the present invention over various time periods in a Duplex package type; and
Figure 11 shows an analysis of impurity levels of 3 embodiments of the present invention over various time periods in a Triplex package type.

### DETAILED DESCRIPTION

As used herein, the term "PK" or "pharmacokinetics" refers to the study of the absorption, distribution, metabolism, and excretion of drugs.

As used herein, the term "mean", when preceding a pharmacokinetic value represents the arithmetic mean value of the pharmacokinetic value taken from a population of patients unless otherwise specified (e.g., geometric mean).

As used herein, the term "Cₘₐₓ" refers to the maximum plasma concentration.

As used herein, the term "C*ₘᵢₙ*" refers to the minimum plasma concentration reached after a drug has been dosed and prior to the administration of a second dose.

As used herein, the term *"Tₘₐₓ"* refers to the time to reach maximum plasma concentration.

As used herein, the term "AUC" refers to the integral of the concentration-time curve.

As used herein, the term "bioavailability" means the rate and extent to which the active drug substance is absorbed from a pharmaceutical dosage form and becomes available at the site of action.

As used herein, the term "bioequivalence" (BE) is the absence of a significant difference in the rate and extent to which the active ingredient becomes available at the site of drug action when administered at the same molar dose under similar conditions in an appropriately designed study. A drug product containing the same active ingredient in the same amount as another drug product, is considered to be bioequivalent to the approved drug product if the rate and extent of absorption do not show a significant difference from the approved drug product, or the extent of absorption does not show a significant difference and any difference in rate is intentional or not medically significant.

The United States bioequivalence criteria is that the 90% confidence interval for the ratio of the means of the AUC₀₋₁₂ and Cₘₐₓ should lie completely within the range 0.80-1.25 for log transformed data. Canada has the same criterion as the United States for AUC₀₋₁₂ but Canadian guidelines require only that the ratio of means for Cₘₐₓ lie within the range 0.80-1.25 (not the confidence interval of the ratio of the means).

Aspects of the present invention are directed to a lozenge comprising nicotine polacrilex and a combination of water soluble synthetic or semi-synthetic non-ionic alkylcellulose polymers having varied viscosities. Applicants have recognized that, surprisingly, the use of a combination of water soluble synthetic or semi-synthetic non-ionic polymers having varied viscosities provides for a lozenge that is bioequivalent to traditional lozenges using naturally occurring polymers and has significant advantages over the traditional lozenges.

Water soluble synthetic or semi-synthetic non-ionic polymers include alkylcelluloses, hydroxyalkylcelluloses, hydroxyalkyl alkylcelluloses, polyalkylene oxides, carboxyalkylcellulose esters methacrylate copolymers; polyvinylalcohol; polyvinylpyrrolidone, copolymers of polyvinylpyrrolidone with vinyl acetate; combinations of polyvinylalcohol and polyvinylpyrrolidone and copolymers of ethylene oxide and propylene oxide. Exemplary alkylcelluloses may include methylcellulose. Exemplary hydroxyalkylcelluloses may include hydroxymethylcellulose, hydroxyethylcellulose, hydroxypropylcellulose and hydroxybutylcellulose. Exemplary hydroxyalkyl alkylcelluloses may include hydroxyethyl methylcellulose and hydroxypropyl methylcellulose; Exemplary polyalkylene oxides may include polyethylene oxide and polypropylene oxide. water soluble synthetic or semi-synthetic non-ionic polymers may also include dextrin, semisynthetic starch, polyhydroxyethylmethacrylate (PHEMA), water soluble nonionic polymethacrylates and their copolymers, modified cellulose, modified polysaccharides, nonionic semisynthetic gums, nonionic polysaccharides and/or mixtures thereof.

According to the invention, the water soluble, synthetic or semi-synthetic non-ionic polymer is alkylcellulose. In certain embodiments, the polymer is an alkylcellulose ether derivative such as hydroxypropyl methylcellulose and hydroxypropyl cellulose. In another embodiment, the polymer is hydroxypropyl methylcellulose. In yet another embodiment, the polymer is hydroxypropylmethyl cellulose (HPCM). In certain embodiments, the polymer has an average partcicle size range of between about 10 and about 100 µm, or between about 20 and about 80 µm, or between about 40 and about 60 µm.

Applicants have recognized that the use of non-ionic polymers appears to increase the bioavailability of the nicotine, especially when the nicotine is in a complex, such as, for example, nicotine polacrilex. Anionic polymers, such as xanthan gum, sodium alginate and calcium polycarbophil, which are used in traditional lozenges, actually consume buffer and bind to cationic nicotine to slow down nicotine absorption through buccal tissues. Nonionic polymers, however, facilitate the nicotine availability without complexing with buffer or nicotine and make nicotine available in protonated form for absorption under buffered microenvironment.

Lozenges of the present invention contain an alkylcellulose polymer having a high viscosity and an alkylcellulose polymer having a low viscosity. In certain embodiments, the polymer having the high viscosity has a viscosity of from about 2,000 cps to about 6,000 cps, or from about 3,000 cps to about 5,000 cps, or from about 3,500 cps to about 5,500 cps. In one embodiment, the high viscosity polymer has a viscosity of about 4,000 cps. Viscosity for the high viscosity polymers was determined using a Brookfield type LV Model, or equivalent.

In certain embodiments, the polymer having the low viscosity has a viscosity of from about 50 cps to about 150 cps, or from about 80 cps to about 100 cps, or from about 90 cps to about 110 cps. In one embodiment, the low viscosity polymer has a viscosity of about 100 cps. Viscosity for the low viscosity polymers was determined using Capillary Viscometer Methods 911.

The amount of high viscosity polymer may be from between about 1% and about 20% by weight, or from between about 2% and about 10% by weight, or from about 3% and about 7% by weight. The amount of low viscosity polymer may be from between about 1% and about 20% by weight, or from between about 2% and about 10% by weight, or from about 3% and about 7% by weight.

The ratio of high viscosity polymer to low viscosity polymer in the lozenge may vary depending upon the desired dissolution characteristics of the lozenge. For example, if a slowly dissolving lozenge is desired, a higher ratio of high viscosity polymer to low viscosity polymer may be desired. If, however, a quickly dissolving lozenge is desired, a lower ratio of high viscosity polymer to low viscosity polymer may be desired. In certain embodiments, the ratio of high viscosity polymer to low viscosity polymer may be between about 1:50 to about 50:1, or between about 1:30 to about 30:1, or between about 1:20 to about 20:1, or between about 1:10 to about 10:1, or between about 1:2 to about 2:1.

It has been recognized that optimization of the ratio of high viscosity polymer to low viscosity polymer results in a lozenge with improved dissolution characteristics. For example, too much high viscosity polymer results in lozenges having highly varied dissolution profiles on a lozenge to lozenge basis. In addition, the amount of high viscosity polymer may not be consistent on an intra-lozenge basis - that is, the high viscosity polymer may not be evenly distributed throughout the lozenge. If only low viscosity polymers are used in a lozenge, nicotine may be released from the lozenge too quickly. To obtain an appropriate release using only low viscosity polymer, a large amount of polymer may be required, resulting in a larger tablet with undesirable textural properties, i.e., the tablet may have a slimy mouth feel.

If, however, a combination of high viscosity polymer and low viscosity polymer are used in the lozenge, lozenge to lozenge dissolution variation can be well controlled. Although not intending to be limited to a single theory, Applicants surmise that the combination of a low and high viscosity polymers in the polymer matrix may provide improved dissolution control because when the tablet is exposed to dissolution media the low viscosity polymer quickly swells and forms a gel layer to control the nicotine release. After the low viscosity polymer forms the gel layer, the high viscosity polymer begins to swell and form a strong gel with the low viscosity polymer to provide uniform drug release from the lozenge.

NRT lozenges of the present invention include the nicotine active nicotine polacrilex. The term "nicotine active" refers to one or more compounds selected from: nicotine; derivatives of nicotine, such as nicotine salts and nicotine complexes; tobacco extract or leaf; any compounds or compositions that produce a similar physiological effect as nicotine, such as lobeline; and mixtures thereof. A variety of nicotine actives are well known in the art and are commercially available. Nicotine actives include nicotine monotartrate, nicotine bitartrate, nicotine hydrochloride, nicotine dihydrochloride, nicotine sulfate, nicotine zinc chloride monohydrate, nicotine salicylate, nicotine oil, nicotine complexed with cyclodextrin, polymer resins such as nicotine polacrilex, and mixtures thereof. The nicotine active may be used in one or more distinct physical forms well known in the art, including free base forms, encapsulated forms, ionized forms and spray-dried forms.

According to the invention, the nicotine active is the nicotine resin complex nicotine polacrilex. In some embodiments, the lozenges contains between about 2 mg and about 50 mg nicotine polacrilex, or from about 5 mg to about 25 mg nicotine polacrilex, or from about 10 mg to about 20 mg nicotine polacrilex. The amount of nicotine in the dosage form (subtracting the amount of resin in the polacrilex complex) may be from about 1 mg to about 10 mg, or from about 2 mg to about 8 mg, or from about 2 mg to about 6 mg. In one embodiment, the lozenge contains about 2 mg nicotine. In another embodiment, the lozenge contains about 4 mg nicotine.

NRT lozenges of the present invention may also contain at least one alkaline buffering agent. Alkaline buffering agents suitable for use in the present invention include, but are not limited to, sodium carbonate, sodium bicarbonate, potassium phosphate, potassium carbonate and potassium bicarbonate. In one embodiment, the buffering agents are selected from potassium bicarbonate, sodium carbonate and mixtures thereof. The total amount of buffer present in the compositions of the present invention may be from about 10 mg to about 50 mg. In one embodiment the total amount of buffer present in the compositions of the present invention is from about 20 mg to about 30 mg. In one embodiment the ratio of nicotine polacrilex to total buffer is from about 3:1 to about 1:3 by total weight, or from about 2:1 to about 1:2 by total weight.

Lozenges of the present invention may also include at least one diluent, at least one excipient selected from the group consisting of taste masking agents, antioxidants, glidants, and colorants, or any combination thereof.

Suitable diluents may include, for example, maltitol, maltose, fructose, glucose, trehalose, sorbitol, sucrose, sugar, mannitol, xylitol, isomalt, dextrose, maltodextrin, dextrates, dextrin, erythritol, lactitol, polydextrose and mixtures thereof. In one embodiment, the diluent is mannitol. In one embodiment, the diluent is present from about 500 mg to about 1,100 mg per lozenge, in another embodiment from about 750 mg to about 1,000 mg per lozenge.

Suitable taste masking agents include, but are not limited to intensive sweetening agents and/or flavorants. Suitable intensive sweetening agents include, but are not limited to, aspartame, acesulfame K, cyclamate and salts thereof, glycyrrhizin and salts thereof, neohesperidine, sucralose, saccharin and salts thereof, thaumatin and mixtures thereof. Suitable flavorants include, but are not limited to, menthol, peppermint, wintergreen, sweet mint, spearmint, vanillin, chocolate, coffee, cinnamon, clove, tobacco, citrus and fruit flavors and mixtures thereof. When present, taste masking agents are present in an amount from about 1 mg to about 50 mg per lozenge, or from about 10 mg to about 20 mg per lozenge. Suitable antioxidants include, but are not limited to sodium benzoate, butyl-hydroxy toluene and tocopherol and its salts. Suitable glidants include, but are not limited to, talc, corn starch, stearic acid, calcium stearate, polyethylene glycol, colloidal silicon dioxide, sodium stearyl fumarate, magnesium stearate, vegetable and mineral oils and mixtures thereof. In one embodiment the glidant is magnesium stearate. Suitable colorants for use herein include any pigments, dyes, lakes or natural food colors that are suitable for food and drug applications, eg. FD&C dyes and lakes.

Lozenges of the present invention may have a total weight per lozenge of between about 100 mg to about 2,000 mg, or between about 500 mg and about 1500 mg, or between about 1,000 mg and about 1,300 mg. In one embodiment the total weight per lozenge is about 1,200 mg.

Lozenges of the present invention may be compressed by traditional tabletting compression techniques. In certain embodiments, the lozenges may be compressed to a hardness of from about 20 N to about 200 N, or from about 30 N to about 150 N, or from about 50 N to about 100 N.

Certain aspects of the present invention are directed to lozenges comprising an intragranular component and an extragranular component. The use of intragranular components for formulations is common in solid dosage forms such as tablets and compressed lozenges. Typically, the intragranular component (or "master granules") is made to improve the processability of a solid dosage form and to reducing friability during transportation and handling. In the absence of an intragranular component, tablets or lozenges where high levels of non-direct compressible diluents are used can be difficult to process or result in a product with high friability. In a typical nicotine lozenge formulation, such as that of the NICORETTE® lozenge, diluents and binding agents are generally granulated together along with buffering agents to form an intragranular component. Active agents, and other optional excipients and flavoring agents, are thereafter blended with the intragranular component, prior to compressing, and make up the "extragranular" component of these traditional lozenge formulations.

Intragranular components may be formed by suitable means such as, for example, slugging, aqueous or non-aqueous wet granulation, fluidized bed granulation, spray drying or roller compaction. In one embodiment, the granulate is formed by a wet granulation process wherein the intragranular ingredients are mixed in a suitable granulator to form a powder blend. Water or a suitable solvent or solvent mixture is added and mixed thoroughly with the powder blend. This process allows the powder blend to become wet and to agglomerate to form granules. The wet granules are then dried in a conventional tray drier and then generally milled and screened to obtain granules with a desired particle size distribution. In another embodiment, the granulate is formed by a fluidized bed granulation process in which the intragranular ingredients are fluidized in a fluid bed drier and then sprayed with water or suitable solvent. The wet granules so formed are dried and are then generally milled and screened to obtain granules with a desired particle size distribution. In another embodiment spray granulation is used as a method to granulate powders to obtain spherical free flowing granules. In a spray granulation operation, the desired intragranular ingredients are suspended in water or suitable solvent. This suspension is sprayed using an atomizer into a spray drier. The droplets so generated by the atomizer are dried to form granules, which are then generally milled and screened to obtain granules with a desired particle size distribution. In yet another embodiment, roller compaction may be used as a method for manufacture of the granulate, where a dry blend of the other desired intragranular ingredients are forced through a pair of rollers held under high pressure, thereby compacting the powder compacts to form wafer like sheets, which are then generally milled and screened to obtain granules with a desired particle size distribution. Small amounts of water can be sprayed on to the powder blend prior to feeding in to the rollers to enhance the binding properties of the ingredients in this process. The granules so obtained by any of the granulation processes described can be further processed to obtain tablets or lozenges.

Presence of polymer in the intragranular component and extragranular component can serve two separate functions. Polymer in the intragranular component may serve as a binder to form the master granules. The intragranular component may include a high viscosity water soluble synthetic or semi-synthetic non-ionic polymer, a low viscosity water soluble synthetic or semi-synthetic non-ionic polymer, or both. In one embodiment, the intragranular component contains both a high viscosity water soluble synthetic or semi-synthetic non-ionic polymer and a low viscosity water soluble synthetic or semi-synthetic non-ionic polymer. The amount of high viscosity water soluble synthetic or semi-synthetic non-ionic polymer may be from between about 1% and about 20% by weight, or from between about 2% and about 10% by weight, or from about 3% and about 7% by weight. The amount of low viscosity water soluble synthetic or semi-synthetic non-ionic polymer may be from between about 1% and about 20% by weight, or from between about 2% and about 10% by weight, or from about 3% and about 7% by weight. In one embodiment, the intragranular component comprises about 5% high viscosity water soluble synthetic or semi-synthetic non-ionic polymer and about 5% low viscosity water soluble synthetic or semi-synthetic non-ionic polymer.

The ratio of high viscosity water soluble synthetic or semi-synthetic non-ionic polymer to low viscosity water soluble synthetic or semi-synthetic non-ionic polymers in the intragranular component may be between about 1:50 to about 50:1, or between about 1:30 to about 30:1, or between about 1:20 to about 20:1, or between about 1:10 to about 10:1, or between about 1:2 to about 2:1.

Presence of polymer in the extragranular component may act as a dissolution modifier. Various dissolution profiles can be achieved by varying the amount and ratios of high viscosity polymer and low viscosity polymers. The extragranular component may include a high viscosity water soluble synthetic or semi-synthetic non-ionic polymer, a low viscosity water soluble synthetic or semi-synthetic non-ionic polymer, or both. In one embodiment, the extragranular component includes a low viscosity water soluble synthetic or semi-synthetic non-ionic polymer.

The amount of high viscosity water soluble synthetic or semi-synthetic non-ionic polymer may be from between about 1% and about 20% by weight, or from between about 2% and about 10% by weight, or from about 3% and about 7% by weight.

The amount of low viscosity water soluble synthetic or semi-synthetic non-ionic polymer may be from between about 1% and about 20% by weight, or from between about 2% and about 10% by weight, or from about 3% and about 7% by weight.

In one embodiment, the extragranular component contains about 2% low viscosity water soluble synthetic or semi-synthetic non-ionic polymer, or about 5% low viscosity water soluble synthetic or semi-synthetic non-ionic polymer, or about 18% low viscosity water soluble synthetic or semi-synthetic non-ionic polymer.

The ratio of high viscosity water soluble synthetic or semi-synthetic non-ionic polymer to low viscosity water soluble synthetic or semi-synthetic non-ionic polymer in the intragranular component may be between about 1:50 to about 50:1, or between about 1:30 to about 30:1, or between about 1:20 to about 20:1, or between about 1:10 to about 10:1, or between about 1:2 to about 2:1.

Nicotine polacrilex may be present in the intragranular component, the extragranular component or both. In one embodiment, nicotine active is present in the extragranular component. Alkaline buffer may also be present in the intragranular component, the extragranular component or both. In one embodiment, alkaline buffer is present in the extragranular component. In another embodiment, alkaline buffer is present in the intragranular component and the extragranular component.

Lozenges of the present invention may have an *in vitro* dissolution profile (as determined by USP Type II apparatus, rotating paddle, with 900 ml of Phosphate buffer at pH 7.4, 37°C set at rotating speed of 75rpm) of:
25 to 50% at 1 hour;
50 to 99% at 3 hours;
75 to 100% at 6 hours.

In other embodiments, lozenges of the present invention may have an *in vitro* dissolution profile (as determined by USP Type II apparatus, rotating paddle, with 900 ml of Phosphate buffer at pH 7.4, 37°C set at rotating speed of 75rpm) of:
30 to 40% at 1 hour;
50 to 70% at 3 hours;
90 to 100% at 6 hours.

In yet other embodiments, lozenges of the present invention may have an *in vitro* dissolution profile (as determined by USP Type I apparatus, basket, Phosphate buffer at pH 7.4, 37°C set at rotating speed of 100rpm) of:
33 to 37% at 1 hour;
65 to 70% at 3 hours;
97 to 100% at 6 hours.

In certain embodiments, lozenges of the present invention may have the following dissolution profile in the oral cavity:
45 to 60% at 15 minutes;
70 to 85% at 30 minutes:
90 to 100% at 60 minutes.

In another embodiment, lozenges of the present invention may have the following dissolution profile in the oral cavity:
50 to 55% at 15 minutes;
75 to 80% at 30 minutes:
95 to 100% at 60 minutes.

In one embodiment, 100% of a lozenge of the present invention is dissolved in the oral cavity in less than about 60 minutes, or in less than about 50 minutes, or in less than about 45 minutes.

In another embodiment, at least about 50% of the lozenge is dissolved in the oral cavity in less than about 30 minutes or in less than about 15 minutes.

A significant advantage of lozenges of the present invention compared to traditional lozenges is that although the dissolution profiles of the current lozenges may vary significantly, these lozenges unexpectedly are still bioequivalent to currently approved and marketed traditional lozenges, such as NICORETTE® or NIQUITIN® or NICABATE® lozenges. This is significant because this provides formulators more leeway in designing dosage forms with specific dissolution profiles that are still bioequivalent to traditional lozenges.

Lozenges of the present invention may be bioequivalent to traditional lozenges such as NICORETTE® Original 4 mg lozenges. Furthermore, it is contemplated that the scope of the invention includes the full breadth of bioequivalence of the data presented in all the figures herein and not be solely limited to actual ratio of the means.

*In-vitro*/*in vivo* Correlation (IV/IVC) is a form of pharmacokinetic modeling to model *in vivo* response as a function of the *in vitro* data and is used as a predictive tool in formulation development. The purpose of IV/IVC is to determine if *in vivo* data can be predicted from *in vitro* data in a highly predictable way. The *in vivo* response is dependent upon the concentration of the drug in plasma, whereas *in vitro* data is determined using a USP dissolution test for the particular drug in question.

If IV/IVC is established, it may then be used to determine the desired *in vitro* profile that would match the observed *in vivo* absorption profile of a predetermined drug. IV/IVC modeling has been conducted on lozenges of the present invention and results of these biostudies provide a good correlation between *in vitro* drug release and *in vivo* drug absorption.

Biologically, the formulations of the present invention, although they may have different dissolution profiles than the reference standard, have AUCs, Cₘₐₓ and Tₘₐₓ, which are the same as (or similar to) the reference standard. In other words, formulations of the current invention are biologically equivalent to the approved reference formulation. Therefore, it is contemplated that the scope of the invention includes the full breadth of bioequivalence of the data presented in all the figures herein and not be solely limited to actual ratio of the means.

In certain embodiments, lozenges of the present invention may have a median time to maximum plasma concentration of nicotine (Tₘₐₓ) from about 1 hour to about 2 hours after administration, or from about 1.2 hours to about 1.7 hours after administration, or from about 1.3 hours to about 1.5 hours after administration.

In certain embodiments, lozenges of the present invention may have a mean plasma concentration (Cₘₐₓ) of nicotine from about 16 ng/ml to about 20 ng /ml, or from about 17 ng/ml to about 19 ng/ml. In other embodiment, lozenges of the present invention may have a mean plasma concentration (Cₘₐₓ) of nicotine between 80% and 125% of the mean plasma concentration (Cₘₐₓ) of 18.67 ng /ml, or may have a mean plasma concentration (Cₘₐₓ) that is bioequivalent to the mean plasma concentration (Cₘₐₓ) of a currently approved nicotine lozenge, such as, for example, a NICORETTE® Original 4mg lozenge.

In certain embodiments, lozenges of the present invention may have a mean Area Under the Curve (AUC₀₋₁₂) of nicotine of between 80 and 100 ng*hr/mL, or between about 85 and about 95 ng*hr/mL. In certain embodiments, lozenges of the present invention may have a mean Area Under the Curve (AUC(₀₋₁₂)) of nicotine of between 80% and 125% of the mean Area Under the Curve (AUC₍₀₋₁₂₎) of 90 ng*hr/mL. Lozenges of the present invention may have a mean Area Under the Curve (AUC₍₀₋₁₂₎) that is bioequivalent to the mean Area Under the Curve (AUC₍₀₋₁₂₎) of a currently approved nicotine lozenge, such as, for example, a NICORETTE® Original 4mg lozenge.

Another advantage of the current lozenges compared to traditional formulations is their ability to remain stable for longer periods of time in less expensive packaging. Nicotine is a moisture sensitive molecule and requires precaution when handling and packaging. Moisture leads to oxidation of nicotine and often results in the generation of oxide impurities when nicotine products are packaged in simple and lower moisture barrier packs such as PVC/PVDC/Duplex as compared to Triplex/Zymax /Alu-Alu or some specially designed desiccant coated HDPE container. High moisture barrier options are available at high cost and contribute to the overall product cost. These costs are prohibitive when attempting to make NRT products available to consumers with less financial means.

Applicants have recognized that lozenges of the present invention are more stable in less expensive packaging than traditional lozenges. For example, aspects of the present invention are directed to lozenges having a stability at a temperature of 40°C and a relative humidity of 75% in a Duplex package for at least 6 months, or for at least 12 months, or for at least 24 months.

Although not intending to be limited to a particular theory, Applicants surmise that the increase in stability of the new lozenges is at least partially attributable to the fact that when Nicotine is exposed to water in the presence of HPMC, HPMC has more affinity toward water uptake, so HPMC will take up the water and prevent nicotine exposure to water. Less exposure to water results in less oxidation and less impurity generation.

Lozenges of the present invention are useful as a tobacco replacement, and as a means to reduce or stop tobacco use. The compositions may be used as a total or partial replacement of tobacco, and may be used concurrently with tobacco as part of a planned tobacco reduction program, e.g., while reducing tobacco usage prior to outright quitting tobacco usage. A user may consume a lozenge of the present invention at set intervals throughout the day as part of a tobacco quit regime. Alternatively, a user may consume a lozenge of the present invention intermittently in response to an acute nicotine craving. In one embodiment a user may consume a lozenge of the present invention at both predetermined intervals as well as intermittently throughout the day to assist with craving relief.

The present disclosure also relates to methods of reducing tobacco usage, comprising administering a composition of the present invention to a person in need thereof. The present invention also relates to a method of reducing nicotine withdrawal symptoms comprising administering the compositions of the present invention to a person in need of such relief. "Need" is intended to include a person's desire to reduce tobacco usage or nicotine withdrawal symptoms, respectively. "Reducing" nicotine withdrawal symptoms or tobacco usage includes eliminating nicotine withdrawal symptoms or tobacco usage.

### EXAMPLES

### Example 1 - Preparation of Nicotine Containing Lozenges

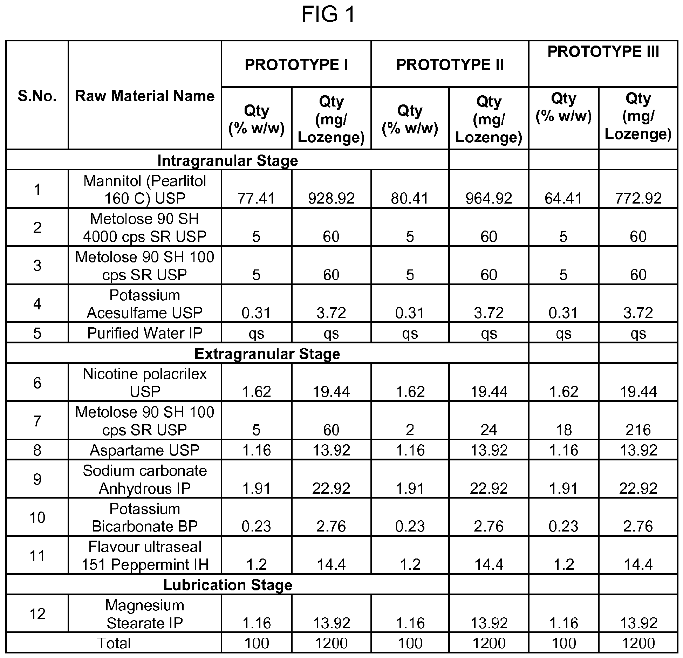

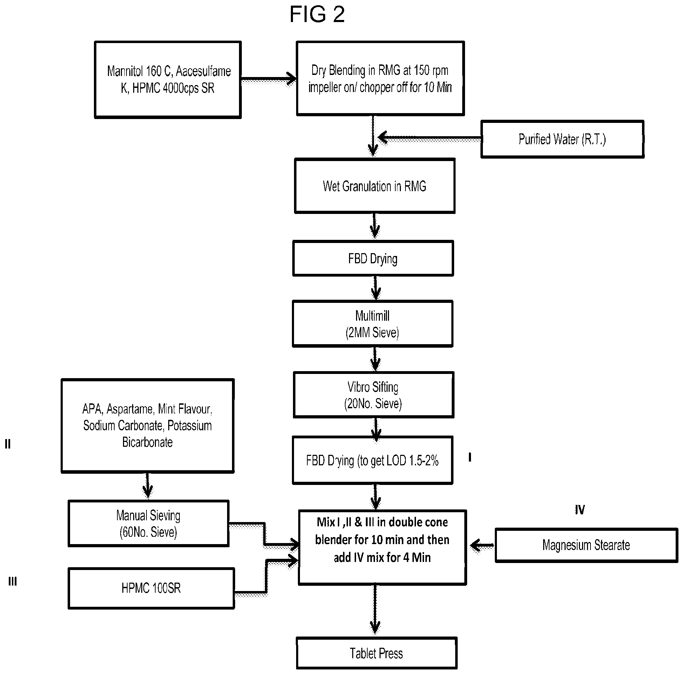

Nicotine lozenges; Prototype I, Prototype II, and Prototype III, having the components of Figure 1 were formed using the process of Figure 2 and as described below:

### Granulation Stage

1. Mannitol was mixed with intragranular portion of HPMC and potassium acesulfame for 10 min at low impeller speed, followed by the addition of purified water and mixed at low chopper and slow impeller to achieve the ampere reading of 15-17 AMP (considered the end point for wet granulation).
2. Post granulation drying in FBD at 50-60°C was performed until the LOD was between 2-2.5% .
3. Granulates were sifted and milled to pass them though 20# ASTM mesh. ***Blending Stage***
4. The extragranular materials i.e. nicotine polacrilex (NPA), buffers, flavors, sweeteners and extragranular portion of HPMC were sifted through 20# ASTM mesh.
5. Intragranular components and extragranular components were blended in double cone blender at 12 rpm for 30 min.

### Lubrication Stage

6. The post blending component was then blended with magnesium stearate for 5 min.

### Compression

8. 1200 mg lozenges were compressed on D tooling tablet press keeping hardness range of 90±20 N where the main compression force was between 15-20kN and precompression force was between 1.5 to 2.5 kN with the friability range of NMT 0.8%.

### Example 2 - Dissolution Profile of New Formulations and Comparison to Reference

The dissolution profiles of the Prototypes prepared in Example 1 were determined and compared to the dissolution profiles of a commercially available Reference (NICORETTE® (original), 4mg nicotine polacrilex lozenge).

The method consisted of a six-hour dissolution test, in pH 7.4 phosphate buffered dissolution media, using USP Apparatus 1 (baskets) at 100 rpm. The prepared samples were analyzed by reverse phase HPLC using a mobile phase of acetonitrile:ammonium hydroxide/sodium perchlorate using a gradient method. Nicotine was quantified by UV detection at 261 nm. Associated calibration was performed through an application of an external standard technique. The Run time for the method was 6 minutes.

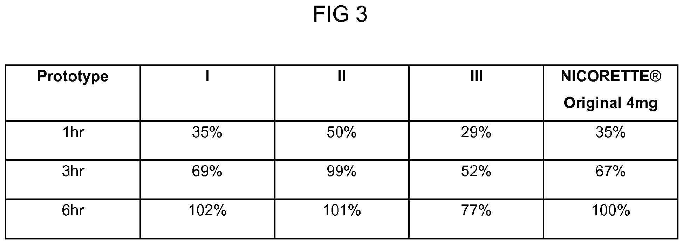

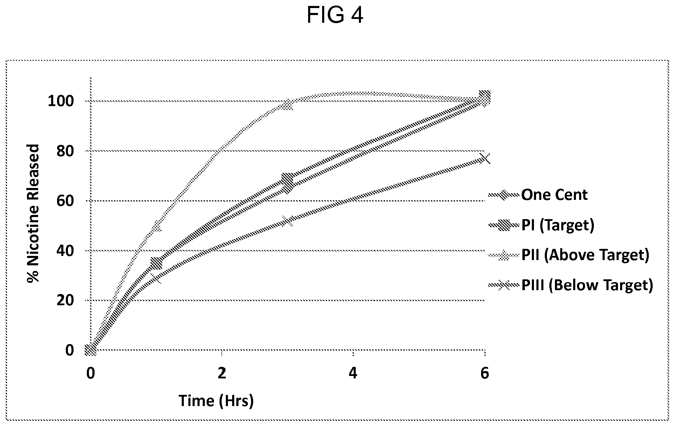

Dissolution data can be seen in Figure 3 and a comparison of the dissolution profile of Prototypes I, II, and III to the NICORETTE® product can be seen in Figure 4.

### Example 3 - A Randomized, Cross-Over, Single Dose Pharmacokinetic Study of Nicotine Lozenges of the Present Invention

### Objectives

### Primary Objective

To compare the AUC₀₋ₜ and Cₘₐₓ of Prototypes I, II, and III to an internationally marketed 4mg nicotine lozenge (NICORETTE® (original) 4mg lozenge).

### Secondary Objectives

To compare the AUC_{0-inf} , tₘₐₓ, Kₑₗ and t_{1/2 o}f Prototypes I, II, and III to an internationally marketed 4mg nicotine lozenge (NICORETTE® (original) 4mg lozenge).

To evaluate safety of the 3 prototypes during the study.

### Design / Methodology

This was a randomized, single center, open label, single dose, four way crossover study in fasted healthy male subjects to compare the pharmacokinetics of nicotine following administration of Prototype I, II, and III lozenges to an internationally marketed 4mg nicotine lozenge (NICORETTE® (original) 4mg lozenge).

Screening procedures were carried out on subjects who consented to participate in the study. Subjects received each of the four study treatments in a randomised order:
- a single dose of 4mg nicotine lozenge (Prototype I)
- a single dose of 4mg nicotine lozenge (Prototype II)
- a single dose of 4mg nicotine lozenge (Prototype III)
- a single dose of 4mg nicotine lozenge (NICORETTE® (original) 4mg lozenge)

The study consisted of a screening visit followed by four study sessions each with 12 hours of blood sampling post-dose. Subjects were confined in the study facility for approximately 50 hours during each study session (for 36 hours pre dosing and for 14 hours post dosing) during which pharmacokinetic (PK) blood samples were obtained. Subjects abstained from smoking during the confinement periods and were subjected to random measurements of expired carbon monoxide (CO) to confirm abstinence.

The four study sessions were separated by at least a 48-hour washout period between doses. Subjects were allowed to go home for one day post collection of all PK blood samples for a particular treatment session. They were asked to report to the site the following evening.

During this period, when the subjects were not at the site, they were allowed to smoke whenever they desired.

Adverse Events (AEs) were recorded from the time of administration of the study product until the end of study visit including the follow-up period.

### Number of subjects (planned and analyzed)

It was planned to randomize 40 subjects in the study. A total of 102 subjects were screened to randomize 40 subjects and 37 subjects completed all study visits. Three subjects did not complete the study due to loss to follow-up (1) and protocol violation (2). Thirty nine (39) subjects were dosed and completed at least one treatment period and were included in safety and PK analysis. PK analysis was based on PP population.

### Main criteria for inclusion

Male subjects aged 18 to 45 years inclusive, who smoked commercially manufactured cigarettes on a daily basis, smoked their first cigarette within 30 minutes after awakening with a smoking history of minimum of twelve months, had a body mass index ranging from 19-27 kg/m², and provided written informed consent participated in the study.

### Study product, dose and mode of administration, batch number

- 4mg nicotine lozenge (Prototype I) was administered orally as a single dose treatment (one lozenge of 4mg) per subject.
- 4mg nicotine lozenge (Prototype II) was administered orally as a single dose treatment (one lozenge of 4mg) per subject.
- 4mg nicotine lozenge (Prototype III) was administered orally as a single dose treatment (one lozenge of 4mg) per subject.

### Reference therapy, dose and mode of administration, batch number

- 4mg nicotine lozenge (internationally marketed 4mg nicotine lozenge, Nicorette® Original) was administered orally as a single dose treatment (one lozenge of 4mg) per subject.

### Duration of treatment

Each subject was administered a single dose of all four treatments.

### Criteria for evaluation

### Efficacy

AUCo-t and Cₘₐₓ was used to compare bioavailability of each prototype to the NICORETTE® (original) 4mg lozenge. The comparisons were made by means of 90% confidence intervals for ratio of the geometric means.

### Safety

Adverse events were used to evaluate safety and tolerability of the study products.

### Statistical methods

A linear mixed effects model was used to analyze the logarithmically transformed (natural log) primary endpoints (baseline adjusted AUCo-t and Cₘₐₓ) using PROC MIXED of SAS. The model included factors for subjects, as a random effect and formulation (treatment) and period as fixed effects. The residual variance from the model was used to construct 90% confidence intervals for the difference between the test treatments and the reference therapy. These were then back-transformed (antilogged) to obtain point estimates and confidence intervals for the ratio of the treatment geometric means.

Tmax was analyzed by non-parametric methods using Wilcoxon Signed Rank test. Median differences among formulations were presented with 95% confidence for the median difference based on the one-sample method by Hodges and Lehman.

AUC_{0-inf} was analyzed in the similar way as Cₘₐₓ and AUCo-t.

### Summary

### Demographic Summary

A total of 39 subjects were included in the safety population. All subjects were male Asians. Reported mean age was 28.7 years (SD=6.44).

### Pharmacokinetic Results

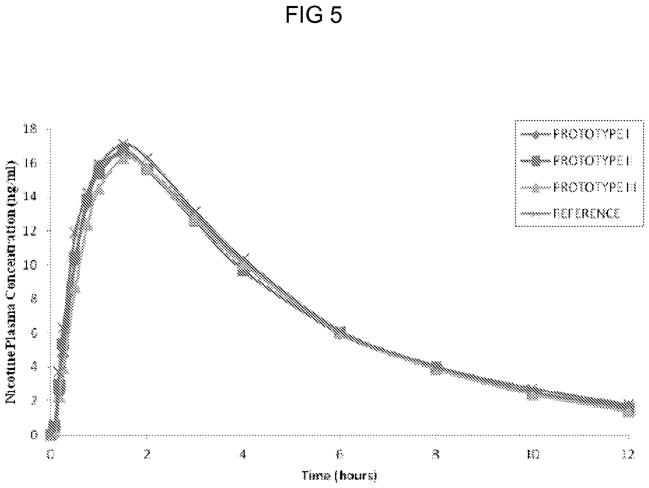

The baseline adjusted mean plasma nicotine concentration versus time curves for all treatments is presented in Figure 5.

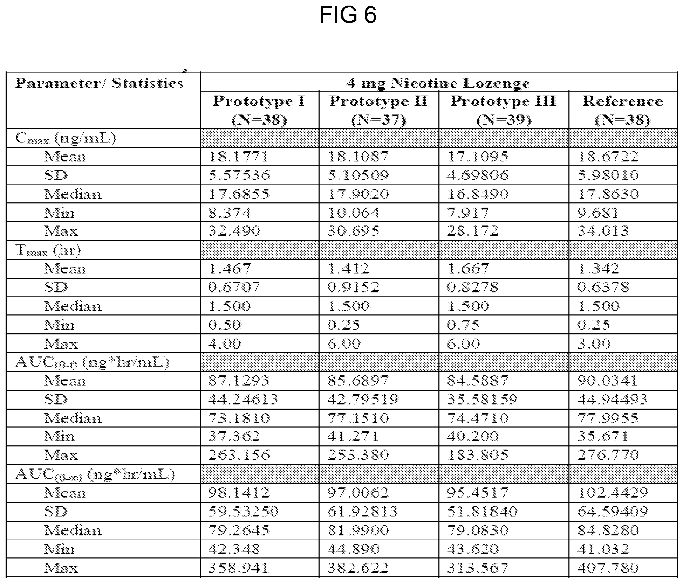

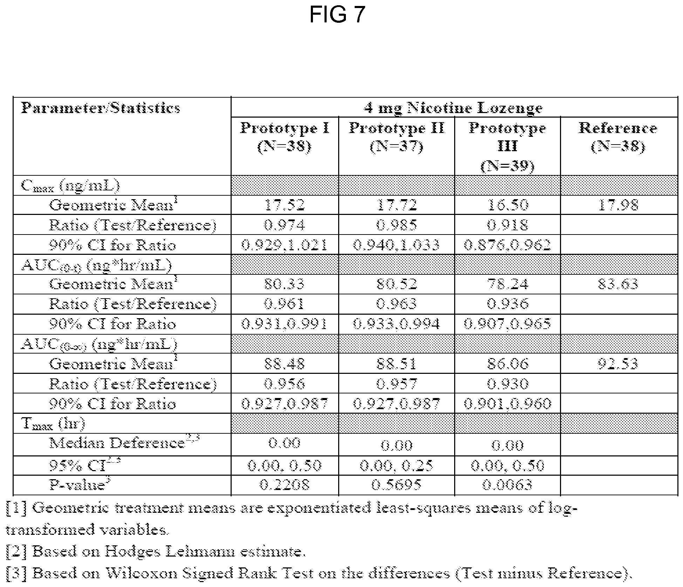

All the pharmacokinetic parameters are summarized in Figure 6. Results of the statistical analysis of AUC_{0-inf}, AUC₀₋ₜₕᵣₛ, Cₘₐₓ and Tmax are given in Figure 7.

Exposure to nicotine for each prototype (I, II and III) 4 mg nicotine lozenge was bioequivalent to reference product (NICORETTE® (original) 4mg lozenge) for Cₘₐₓ, AUCo-t and AUC_{0-∞} with 90% confidence intervals (CIs), all being within the range 0.80 to 1.25 (Figure 7).

Time to reach maximum nicotine plasma concentration (Tₘₐₓ) was significantly greater (p=0.0063) for Prototype III as compared to reference product, whereas no statistical significance observed for Prototype I and II as compared to reference product. Each prototype (I, II and III) and reference product achieved maximum nicotine plasma concentration at 1.5 hours (median). The statistically significant result for Tₘₐₓ with prototype III was due to the fact that maximum nicotine plasma concentration was reached later in majority of the subjects receiving prototype III.

### Safety Results

There was no adverse event reported in this study. All the treatments were well tolerated.

### Conclusions

The three test prototype 4 mg nicotine lozenges were bioequivalent to the NICORETTE® 4mg lozenge (US marketed) based on AUC (o-t) and Cₘₐₓ The three prototype nicotine lozenges were well tolerated in this study.

### Example 4 - Package Stability of Lozenges

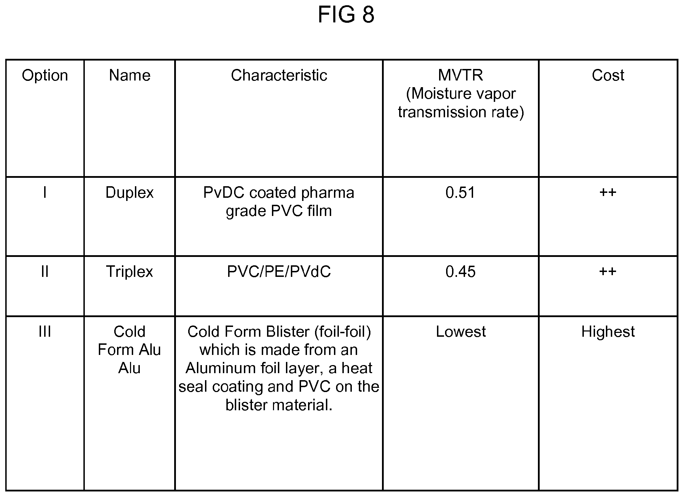

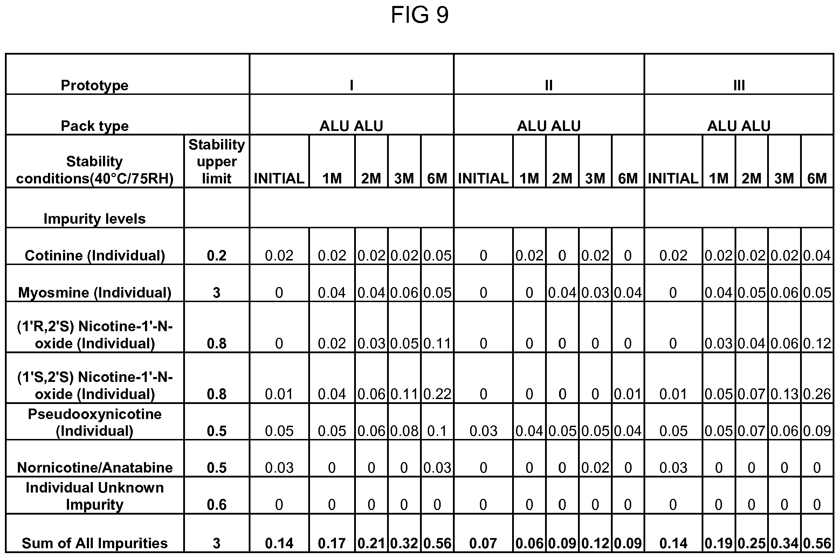

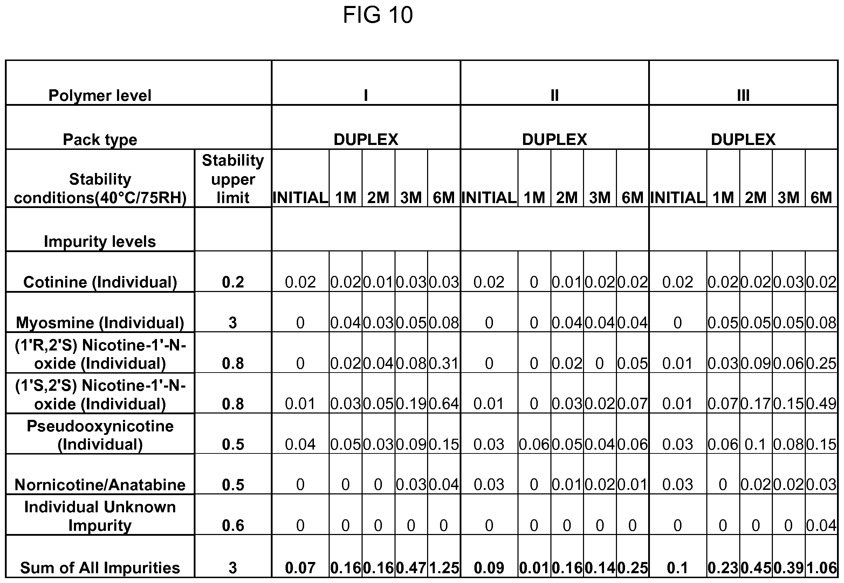

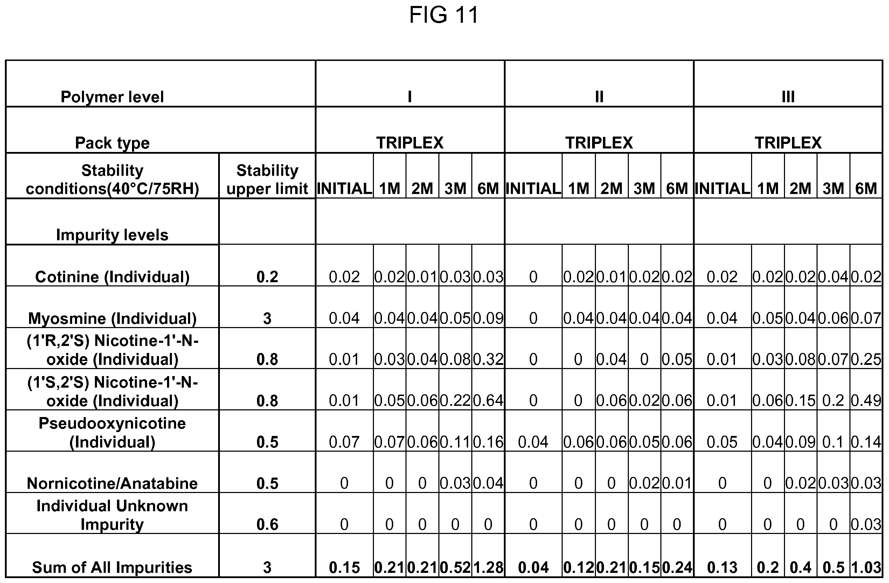

Prototypes I, II, and III were stored in the packaging options shown in Figure 8 at 40°C and 75% relative humidity. At various time points, samples were withdrawn from the packaging and analyzed for the degradation products (impurities). Determination of degradation products was carried out by reversed phase HPLC. The mobile phase was a 15:85 acetonitrile:potassium phosphate/strontium nitrate buffer. The nicotine degradation products were quantified by external standard method using a UV detector at 261 nm. All the related substances i.e. cotinine, myosmine, (1'S, 2'S)-Nicotine-1'-N-oxide, (1'R, 2'S)-Nicotine-1'-N-oxide, pseudooxynicotine, and nornicotine/anatabine were well separated from each other and other excipients. The method is stability indicating and completely validated for all parameters like specificity, linearity, precision, accuracy, robustness and solution stability. The LOD/LOQ values for all the compounds were also established. Stability results for ALU ALU are shown in Figure 9, for Duplex are shown in Figure 10, and for Triplex in Figure 11. It was observed during stability studies that HPMC plays a role in controlling the impurity generation in low moisture barrier pack option such as Duplex. When the amount of HPMC in the formulation was increased from 3% to 18% the impurity generation in low moisture barrier pack options decreased further.

## Claims

1. A nicotine lozenge for oral administration comprising:
nicotine polacrilex;
at least one high viscosity, water soluble, synthetic or semi synthetic, non-ionic polymer, which is an alkylcellulose polymer having a viscosity of between about 2,000 cps to about 6,000 cps as measured by Brookfield type LV Model; and
at least one low viscosity, water soluble, synthetic or semi synthetic, non-ionic polymer, which is an alkylcellulose polymer having a viscosity of between about 50 cps and about 150 cps as measured by Capillary Viscometer Methods 911.

2. The lozenge of claim 1, wherein the ratio of high viscosity alkylcellulose polymer to low viscosity alkylcellulose polymer is between about 1:50 and about 50:1.

3. The lozenge of claim 2, wherein the ratio of high viscosity alkylcellulose polymer to low viscosity alkylcellulose polymer is between about 1 :2 and about 2: 1.

4. The lozenge of claim 1, further comprising at least one alkaline buffering agent.

5. The lozenge of claim 1, wherein the alkylcellulose polymer is an hydroxyalkyl alkylcellulose which is hydroxypropylmethylcellulose.

6. The lozenge of claim 1, further comprising at least one dissolution modifier.

7. The lozenge of claim 1, further comprising at least one diluent.

## Patentansprüche

1. Nikotin-Pastille zur oralen Verabreichung, umfassend
Nikotin-Polacrilex;
mindestens ein hochviskoses, wasserlösliches, synthetisches oder halbsynthetisches, nichtionisches Polymer, bei dem es sich um ein Alkylcellulose-Polymer mit einer Viskosität zwischen etwa 2000 cps bis etwa 6000 cps, gemessen nach dem Brookfield-Typ LV-Modell, handelt; und
mindestens ein niedrigviskoses, wasserlösliches, synthetisches oder halbsynthetisches, nichtionisches Polymer, bei dem es sich um ein Alkylcellulose-Polymer mit einer Viskosität zwischen etwa 50 cps und etwa 150 cps, gemessen nach der Kapillarviskosimeter-Methode 911, handelt.

2. Pastille gemäß Anspruch 1, wobei das Verhältnis von hochviskosem Alkylcellulose-Polymer zu niedrigviskosem Alkylcellulose-Polymer zwischen etwa 1:50 und etwa 50:1 liegt.

3. Pastille gemäß Anspruch 2, wobei das Verhältnis von hochviskosem Alkylcellulose-Polymer zu niedrigviskosem Alkylcellulose-Polymer zwischen etwa 1:2 und etwa 2:1 liegt.

4. Pastille gemäß Anspruch 1, die ferner mindestens ein alkalisches Puffermittel umfasst.

5. Pastille gemäß Anspruch 1, wobei es sich bei dem Alkylcellulose-Polymer um eine Hydroxyalkylalkylcellulose handelt, bei der es sich um Hydroxypropylmethylcellulose handelt.

6. Pastille gemäß Anspruch 1, die ferner mindestens ein Auflösungsmodifizierungsmittel umfasst.

7. Pastille gemäß Anspruch 1, die ferner mindestens ein Verdünnungsmittel umfasst.

## Revendications

1. Pastille de nicotine destinée à une administration orale comprenant :
du polacrilex de nicotine ;
au moins un polymère non ionique synthétique ou semi-synthétique soluble dans l'eau, à haute viscosité, qui est un polymère d'alkylcellulose ayant une viscosité comprise entre environ 2 000 cps et environ 6 000 cps telle que mesurée par un modèle LV de type Brookfield ; et
au moins un polymère non ionique synthétique ou semi-synthétique soluble dans l'eau, à faible viscosité, qui est un polymère d'alkylcellulose ayant une viscosité comprise entre environ 50 cps et environ 150 cps telle que mesurée par les méthodes 911 avec un viscosimètre capillaire.

2. Pastille selon la revendication 1, dans laquelle le rapport du polymère d'alkylcellulose à haute viscosité sur le polymère d'alkylcellulose à faible viscosité est compris entre environ 1:50 et environ 50:1.

3. Pastille selon la revendication 2, dans laquelle le rapport du polymère d'alkylcellulose à haute viscosité sur le polymère d'alkylcellulose à faible viscosité est compris entre environ 1:2 et environ 2:1.

4. Pastille selon la revendication 1, comprenant en outre au moins un agent tampon alcalin.

5. Pastille selon la revendication 1, dans laquelle le polymère d'alkylcellulose est une alkylcellulose d'hydroxyalkyle qui est une hydroxypropylméthylcellulose.

6. Pastille selon la revendication 1, comprenant en outre au moins un modificateur de dissolution.

7. Pastille selon la revendication 1, comprenant en outre au moins un diluant.
